# EUROPÄISCHE PATENTSCHRIFT

(11) **EP 3 308 696 B1**
(45) Veröffentlichungstag und Bekanntmachung des Hinweises auf die Patenterteilung: **23.09.2020**
(21) Anmeldenummer: 16193856.8
(22) Anmeldetag: 14.10.2016
(51) Int. Cl.: A61B 5/00, A61B 5/01

(54) **TEMPERATURSONDE ZUR EINFÜHRUNG IN DEN ÖSOPHAGUS UND ZUR ERFASSUNG EINER TEMPERATUR AN DER SCHLEIMHAUTOBERFLÄCHE DES ÖSOPHAGUS**
TEMPERATURE SENSOR FOR INTRODUCTION INTO THE ESOPHAGUS AND FOR DETERMINING A TEMPERATURE ON THE SURFACE OF THE MUCOSA OF THE ESOPHAGUS
SONDE DE TEMPÉRATURE À INTRODUIRE DANS UN SOPHAGE ET DE DÉTECTION D'UNE TEMPÉRATURE SUR LA SURFACE DE MUQUEUSE DE L' SOPHAGE

(43) Veröffentlichungstag der Anmeldung: 18.04.2018
(73) Patentinhaber: Vanguard AG, 12623 Berlin (DE)
(72) Erfinder: Antz, Matthias, 27777 Ganderkesee (DE); Schrödel, Robert, 12099 Berlin (DE); Borgwardt, Jörg, 12099 Berlin (DE)
(74) Vertreter: LS-MP von Puttkamer Berngruber Loth Spuhler

(56) Entgegenhaltungen:
- DE-U1-202010 012 761
- US-A1- 2006 106 375
- US-A1- 2010 317 956
- US-A1- 2013 211 282
- US-A1- 2013 289 369
- US-A1- 2016 029 897

## Beschreibung

Die Erfindung betrifft eine Temperatursonde zur nasalen oder oralen Einführung in den Ösophagus (Speiseröhre) eines menschlichen oder tierischen Körpers zur Temperaturmessung an der Schleimhautoberfläche des Ösophagus. Sie dient der Überwachung und dem Schutz von Gewebe während einer Katheterablation in dem menschlichen und/oder tierischen Körper.

Eine Katheterablation kommt heutzutage bei vielen verschiedenen Herzrhythmusstörungen zur Anwendung. Sie wird bei symptomatischen Tachykardien (Herzrasen) angewandt, die die Betroffenen belasten oder gefährden, ihre Lebensqualität oder Arbeitsfähigkeit einschränken oder wenn die Rhythmusstörung nicht befriedigend medikamentös behandelt werden kann. Dazu zählen beispielsweise Tachykardien, die durch eine zusätzliche Leitungsbahn im Herzen bedingt sind, wie beim WPW-Syndrom. Aber auch Vorhof-Rhythmusstörungen (z.B. Vorhofflimmern oder Vorhofflattern) und Herzkammer-Rhythmusstörungen (z.B. Extraschläge oder Tachykardien) können durch eine Katheterablation behandelt werden. Die Erfolgsraten liegen je nach der Art der zu behandelnden Herzrhythmusstörung zwischen 70 und 99 Prozent.
Die häufigste Herzrhythmusstörung ist das Vorhofflimmern, das bei 1 - 2 % der Bevölkerung vorkommt. Behandlungen zielen auf die Wiederherstellung und den Erhalt des Sinusrhythmus. Die Katheterablation, bei welcher spezielle Katheter, wie Radiofrequenz- (RF), Kryo- oder Laserablationskatheter zum Einsatz kommen, ermöglicht, bestimmte Gewebebereiche im Vorhof und den Einmündungen (Ostien) der Lungenvenen zu veröden, um eine ungewünschte Erregungsleitung zu unterdrücken. Hierzu werden zunächst Katheter zum Mapping der Herzwand in die Vorhöfe eingeführt und anschließend an die Lokalisation des zu behandelnden Gewebes vorgeführt wo dann dessen Verödung erfolgt.

Bei der Ablation mittels RF-Strom wird ein RF-Katheter über große Blutgefäße in das Herz eingebracht und die Wärmeentwicklung des Hochfrequenzstroms bewirkt eine thermische Schädigung des Gewebes. Zur Echtzeitdarstellung der Position des Ablationskatheters innerhalb der Herzanatomie werden bei der Ablation mit RF-Strom neben der Röntgendurchleuchtung oft auch sogenannte dreidimensionale Mappingsysteme eingesetzt. Diese Mappingsysteme bestehen z.B. aus einem Positionssensor im Ablationskatheter und einem aus mehreren Sendern bestehenden Lokalisierungsgerät, welches sich unterhalb des Patienten befindet.

Eine weitere Ablationsart ist die Kryoablation, bei welcher ein flüssiges Kühlmittel, wie beispielsweise Lachgas, in die hohle Spitze des Ablationskatheters, etwa einem dort befindlichen Ballon, geleitet wird, wo es verdampft und dem umgebenen Gewebe thermische Energie entzieht und es auf diese Weise thermisch schädigt. Die Positionskontrolle erfolgt beispielsweise über Röntgenkontrolle während der Platzierung des Katheters und der im Anschluss an die Platzierung gekühlte Ballon verhindert ein Verrutschen des Katheters aufgrund der Haftung am Gewebe.

Eine dritte Ablationsart stellt die Laserablation dar, bei welcher die Verödung des Gewebes über einen im Ballonkatheter integrierten Laser bewirkt wird. In den Katheterschaft ist ein Endoskop eingearbeitet, welches dem Anwender ermöglicht durch den Ballon hindurch die Ablationsstelle zu beobachten. Da Blut die Sicht stören und die Laserenergie absorbieren würde, wird der Ballon in den Lungenveneneingang vorgeschoben und durch leichten Druck in Richtung Gefäßwand das Blut am Lungenveneneingang verdrängt, wodurch eine freie Sicht auf den Lungenveneneingang und somit eine unter Sicht erfolgende Behandlung mittels Laser ermöglicht wird.

Die thermische Verödung von Gewebe birgt jedoch das Risiko, umliegende Gewebeareale irreparabel zu schädigen. Bei der Therapie von Vorhofflimmern kann die Ablation an der posterioren Wand des linken Vorhofs beispielsweise die Ösophaguswand verletzen wodurch sich eine Fistel zwischen dem Ösophagus und dem Vorhof bilden kann, insbesondere dann wenn die Ablation in der Nähe des dem Epikard anliegenden Ösophagus durchgeführt wird. Das Risiko einer Schädigung des Ösophagus stellt ein nicht zu unterschätzendes Komplikationsrisiko des Eingriffs dar.

Es ist demzufolge notwendig während der Ablation die Temperatur im umliegenden Gewebe (insbesondere im Ösophagus) zu überwachen. Beim Überschreiten einer definierten Maximaltemperatur, im Falle einer Ablation von Hochfrequenzstrom oder Laserablation, sowie beim Unterschreiten einer definierten Minimaltemperatur, im Falle einer Kryoablation, kann dann die Ablation gestoppt bzw. die Leistungsabgabe verringert werden, um thermische Schädigungen des Ösophagus zu verhindern. Bisherige Lösungen weisen jedoch erhebliche Nachteile auf.

Aus DE 10 2006 044 659 A1 ist z.B. eine Temperatursonde zur Einführung in den menschlichen oder tierischen Körper, insbesondere des Ösophagus, bekannt. Diese weist einen Ballonkatheter auf, auf dessen Außenseite mehrere, fest mit dem Ballon verbundene, Temperatur- und Positionssensoren angeordnet sein können. Das Befüllen des Ballons sorgt für den erforderlichen Wandkontakt, so dass die Sensoren an die Innenseite des Ösophagus gepresst werden und so die Temperatur an der Ösophaguswand gemessen werden kann. Diese wird dazu verwendet, um geeignete Maßnahmen zum Schutz des Ösophagus, wie die Manipulation von dessen Lage im Körper des Patienten, einzuleiten. Hierfür kann der beschriebene Katheter über einen Seilzug bewegt werden, um den Ösophagus vom Herzen weg zu bewegen. WO 2013064132 A2 beschreibt einen mehrpoligen Ösophaguskatheter zur Überwachung, Stimulation und Kardioversion des Herzens bei Vorhofflimmern, welcher über einen einseitig angeordneten Ballon, Temperatursensoren und Elektroden zur Stimulation verfügt. Der einseitig, ballig angeordnete Ballon dient dazu, die Elektroden und Temperatursensoren auf der ballonfreien Seite des Katheters gegen die Ösophaguswand zu drücken. WO 2013/120043 A1 beschreibt ebenfalls ein Gerät zur Überwachung der Temperaturänderungen im Ösophagus während der Ablation am Herzen. Auch hier wird der zur Messung erforderliche Wandkontakt über eine Hilfsstruktur, wie etwa einem Ballon, erzielt. Die Temperaturmessung erfolgt über die Temperaturänderung in einem Flüssigkeitskreislauf, welcher den Ballon umgibt und Verbindung zu einer Auswerteeinheit besitzt. Hierzu wird ein Fluid mit bekannter Temperatur in den Kreislauf um den Ballon gepumpt und die Temperatur des ausgeworfenen Fluids gemessen. Aus dem Verlauf der Differenz der beiden Temperaturen kann auf eine lokale Temperaturerhöhung oder -minderung im Messbereich geschlossen werden.

US-2013/211282-A1, US-2010/317956-A1, US-2013/289369-A1, DE-202010012761-U1, US-2006/106375-A1, und US-2016/029897-A1 zeigen weitere Temperatursonden.

Viele der bisher eingesetzten Vorrichtungen zur Temperaturmessung über Gewebekontakt üben während der Ablation Kräfte auf den Ösophagus aus, um die Temperatursensoren an der jeweiligen Position zu halten. Dies kann zu einer Verschiebung des Ösophagus in Richtung der Ablationsstelle führen, was das Risiko einer Schädigung des Ösophagusgewebes erhöht. Neben o.g. Nachteil, dass Druck ausgeübt werden muss, führt, wenn die Anpresskraft der Temperatursensoren über einen befüllbaren Ballon erzeugt wird, dies außerdem zu einem teilweisen oder vollständigen Verschluss des Ösophagus, sowie zu einer Dehnung des Ösophagus-Querschnitts. Die Messung der Temperaturverschiebung eines Fluids zur Erfassung der Ösophagustemperatur hat zusätzlich den Nachteil, dass kleine, lokale Temperaturänderungen nur zu sehr kleinen Signaländerungen führen, da der durch die Temperaturerhöhung hervorgerufene Wärmestrom im Vergleich zum Wärmespeicher des Fluids sehr gering ist.

Weiterhin ist in US 20080215047 A1 ein Ösophaguskatheter beschrieben, der über mindestens zwei Temperatursensoren verfügt, welche sich fixiert auf dem Katheterschaft zwischen zwei Positionsmarkern befinden. Und US 2013006139 A1 beschreibt einen Ösophaguskatheter zur Überwachung der Temperatur im Ösophagus, welcher eine atraumatische "J"-förmige Spitze aufweist und periodisch angeordnete, fest auf dem Katheterschaft fixierte Temperatursensoren. Zwischen jeweils zwei benachbarten Temperatursensoren sind Elektroden gleichmäßig angeordnet, welche der räumlichen Darstellung des Ösophagus dienen.

Ein Nachteil der kontaktfreien Messung besteht darin, dass das benötigte Trägersystem, wie etwa ein Endoskop, sehr starr ist. Dies kann anatomiebedingt zu einer Verschiebung des Ösophagus führen, wenn beispielsweise das Endoskop, als Trägersystem Kraft auf die Wand des Ösophagus ausübt. Ein weiterer Nachteil ist, dass nicht sichergestellt ist, dass alle relevanten Bereiche des Ösophagus bei der Temperaturmessung erfasst werden. Der Ösophagus ist im Querschnitt nicht exakt rund sondern nimmt vielmehr Querschnittsformen zwischen rund und sternförmig-eingestülpt an, wodurch bestimmte Areale, insbesondere im Bereich der kleinsten Radien, durch die kontaktfreie Temperaturmessung nicht erfasst werden können, da sie vom umliegenden Gewebe verdeckt werden.

Ein zusätzlicher Nachteil bestehender Vorrichtungen zur Temperaturmessung im Ösophagus ist in der ungenügenden räumlichen und zeitlichen Auflösung der Temperaturverteilung zu sehen. Darüber hinaus kann nicht sichergestellt werden, dass die Temperatur im relevanten Bereich des Ösophagus erfasst wird, unter anderem weil die Lage der geringen Anzahl an Temperatursensoren nicht exakt bekannt ist. Weiterhin müsste bereits bei der Positionierung der Temperatursonde feststehen, in welchem eng begrenzten Bereich die Temperaturerhöhung oder -unterschreitung erwartet wird, so dass die Sensoren ausgerichtet werden können. Den Bereich der Temperaturänderung genau vorauszusagen, ist, beispielsweise aufgrund von unterschiedlicher Wärmeleitung in den einzelnen Geweben, nicht möglich. Bisher wird nur der Bereich, welcher der Ablationsstelle am nächsten liegt, überwacht. Eine komplette Erfassung der Temperatur der gesamten Oberfläche eines Ösophagusabschnitts wird aufgrund der obengenannten Nachteile nur unzureichend oder gar nicht sichergestellt. Die Einflüsse unterschiedlich ausgebildeter Ösophagusquerschnitte werden bei bekannten Vorrichtungen nur unzureichend berücksichtigt, insbesondere bei sternförmig-eingestülpten Querschnittsformen.

Zudem können RF-Ablationen im linken Vorhof zu thermischen Schädigungen des Ösophagusgewebes im Bereich der Sensoren der Temperatursonde führen (wahrscheinlich durch ungewünschten Anstieg der Stromdichte im Bereich der großen Metallelektroden herkömmlicher Temperatursonden).

Der Erfindung liegt daher die Aufgabe zugrunde eine Vorrichtung zur Temperaturmessung im Ösophagus zu entwickeln, welche oben genannte Nachteile nicht aufweist. Insbesondere liegt die Aufgabe darin, eine Vorrichtung zur Temperaturmessung bereitzustellen, die keine oder nur minimale Kräfte auf die Ösophaguswand ausübt, um so eine Verschiebung des Ösophagus zur Ablationsstelle hin zu vermeiden. Weiterhin sollte die Vorrichtung möglichst die gesamte Ösophagusoberfläche erfassen und eine hinreichend hohe räumliche Auflösung gewährleisten. Thermische Schädigungen des Ösophagusgewebes im Bereich der Sensoren z.B. bei RF-Ablationen sollen vermieden werden.

Die Aufgabe der Erfindung kann durch eine Temperatursonde mit den Merkmalen des Anspruchs 1 gelöst werden. Die Unteransprüche stellen Vorzugsvarianten dar. Erfindungsgemäß wird eine Temperatursonde bereitgestellt, die mindestens zwei flexible flächige Sensor-Array-Segmente aufweist, die um einen Katheter rotationssymmetrisch angeordnet sind. Ihre Flächen verjüngen sich zum distalen Ende des Katheters. So bilden sie Anschlussbereiche über die sie am distalen Ende mit dem Katheter verbunden sind. Diese flexiblen flächigen Sensor-Array-Segmente enthalten jeweils eine oder mehrere Temperatursensoren, die über Leiterbahnen miteinander verbunden sind. Dabei sind die Sensoren so auf den flächigen Sensor-Segmenten angeordnet, dass sie nach Einführen der Sonde in den Ösophagus mit der Schleimhautoberfläche des Ösophagus funktional in Kontakt treten, und so über die Temperatursensoren eine Temperatur der Schleimhautoberfläche erfasst werden kann.

Die mindestens zwei flächigen Sensor-Array-Segmente bestehen aus einem weichen Trägermaterial, auf dem eine Vielzahl von Temperatursensoren angeordnet ist, vorzugsweise mindestens 10 Sensoren/Segment. Diese flexiblen Sensor-Array-Segmente gestatten ein Anlegen der Sensoren an die Oberfläche des Ösophagus, mit z.B. runden bis sternförmig-eingestülpten Querschnittsformen, und ermöglichen so Änderungen der Oberflächenkontur, z.B. durch eine Lageänderung des Patienten oder aufgrund von Peristaltik, zu folgen, ohne dass dazu die Sensoren erneut an die Schleimhaut gedrückt werden müssen oder diese den Kontakt zu dieser verlieren.

Die Temperatursonde ist derart gestaltet, dass die Kontaktkraft auf umliegendes Gewebe reduziert ist. Die Temperatursensoren auf den flexiblen flächigen Segmenten, die rotationssymmetrisch um den Katheterschaft in Form eines Sensor-Arrays angebracht sind, ermöglichen so eine Temperaturerfassung nahezu über die gesamte Oberfläche eines definierten Ösophagusabschnittes. Aus den Messwerten werden die Temperaturgrenzwerte ermittelt und an ein Peripheriegerät gesendet. Das Peripheriegerät kann die aktuellen Temperaturgrenzwerte anzeigen und die Ablation automatisch unterbrechen oder die Ablationsenergie reduzieren, um eine Schädigung des Gewebes zu verhindern.

Sowohl Sensor-Array-Segmente als auch Katheter sind aus äußerst biegsamen und weichen Materialien. Sie bestehen bevorzugt aus thermoplastischen Elastomeren (TPE), wie thermoplastischen Copolyamiden, z. B. PEBAX, thermoplastischem Polyesterelastomeren/ thermoplastischen Copolyestern, z. B. Keyflex, thermoplastischen Elastomeren auf Olefinbasis, vorwiegend PP/EPDM, z. B. Santoprene, Styrol-Blockcopolymere (SBS, SEBS, SEPS, SEEPS und MBS), z. B. Kraton, Septon, Styroflex, Thermolast oder Saxomer, thermoplastischen Elastomeren auf Urethanbasis, z. B. Elastollan oder Desmopan, Texin, Utechlan, vernetzten thermoplastischen Elastomeren auf Olefinbasis, vorwiegend PP/EPDM, z. B. Sarlink, Forprene.

Vorzugsweise sind die flexiblen, flächigen Sensor-Array-Segmente derart gestaltet, dass sie im Ruhezustand um den Katheterschaft überlappend gefaltet vorliegen. Nach Einführen in den Ösophagus können sie dann, bevorzugt teilweise überlappend vorliegend, die ganze Oberfläche eines definierten Ösophagusabschnitts abdecken. Auf diese Weise können Aufweitungen des Ösophagusquerschnitts nicht zu Sensorschatten, aufgrund von nicht mit Sensoren abgedeckten Bereichen der Ösophagusoberfläche, führen.

Die erfindungsgemäße Temperatursonde hat den großen Vorteil, dass zur Temperaturüberwachung, z.B. während einer Katheterablation an Stellen des linken Herz-Vorhofs zur Wiederherstellung des Sinusrhythmus, keine Anpresskräfte, z.B. durch einen Stützballon, erforderlich sind. Somit liefert die Temperatursonde nicht nur Temperaturinformationen über die gesamte Oberfläche eines Ösophagusabschnitts, ohne das Risiko von nicht erfassten Bereichen, sondern schließt auch das Risiko aus, den Abstand zwischen Ablationsort und Ösophagus aufgrund von Anpresskräften der Sensoren zu reduzieren.

Da die Temperatursonde bevorzugt aus weichen Materialien ist, die Biegsamkeit gestatten, kann sie trans-nasal oder trans-oral in den Ösophagus eingeführt werden. Insbesondere die Oberfläche der Temperatursonde zur Temperaturmessung im Ösophagus ist so beschaffen, dass sie leicht vom Patienten heruntergeschluckt oder leicht über den nasalen oder oralen Zugang beim Patienten in den Ösophagus und in Richtung Magen vorgeschoben werden kann.

Vorteilhaft ist hier insbesondere, dass die Temperatursonde eingeführt werden kann, unabhängig davon, ob der Patient wach, lokal betäubt, sediert oder vollnarkotisiert ist. Schluckbewegungen des Patienten können die Einführung der Sonde in den Ösophagus erleichtern.

Erfindungsgemäß weisen die flächigen Sensor-Array-Segmente vorzugsweise jeweils distal und proximal röntgendichte (röntgensichtbare) schmale Markerbereiche auf. Zwischen diesen Markern sind die Temperatursensoren angeordnet. Die Temperatursensoren können in den Sensor-Array-Segmenten vom proximalen zum distalen Marker mäandernd oder in Reihe(n) aufeinander folgend angeordnet sein. Vorzugsweise weist ein Segment mindestens 10 und maximal 40 Temperatursensoren auf, besonders bevorzugt zwischen 10 und 30 Temperatursensoren. Durch die Sensoren zwischen proximalem und distalem Marker wird ein Messbereich Iₘ definiert. Die Sensor-Array-Segmente sind in Richtung Katheterspitze vorzugsweise am Beginn der sich verjüngenden Fläche mit dem einen röntgendichten Marker versehen. Am proximalen Ende zwischen Markerbereich und Segmentende (Verbindungsstelle zum Katheter) kann sich in der Regel eine Übergangszone von ca. 1 bis 5 mm befinden.

Die erfindungsgemäße Temperatursonde kann im Übrigen die röntgendichte Marker auf den flexiblen Sensor-Array-Segmenten und/oder dem Katheter aufweisen. Die röntgensichtbaren Marker grenzen den Bereich ab, zwischen denen die Temperaturmessung erfolgt. Insbesondere die Marker auf den Sensor-Array-Segmenten zeigen die Lage der einzelnen Sensorbereiche.

Besonders bevorzugt weist die Temperatursonde vier bis sechs flexible Sensor-Array-Segmente am Katheterschaft angeordnet auf, vorzugsweise sechs. Dabei sind die einzelnen flächigen Segmente derart gestaltet, dass sie ggf. übereinander gefaltet um den Katheterschaft liegen und sich nach dem Einführen in den Ösophagus zumindest mit einem Teil ihrer Fläche überlappen. So können sich die flexiblen Sensor-Array-Segmente an Änderungen im Ösophagus, wie etwa Durchmesseränderungen, flexibel anpassen, sodass Sensorschatten aufgrund nicht bedeckter Ösophagusabschnitte minimiert werden können.

Jedes Segment weist den Bereich Iₘ von Marker zu Marker auf und besitzt innerhalb diesem den Messbereich mit einer bevorzugten Länge von ca. 200-250 mm, vorzugsweise ca. 240 mm. Des Weiteren ist ein Segment vorzugsweise zwischen ca. 19 und 23 mm breit, bevorzugt ca. 20-21 mm. Es weist weiterhin eine bevorzugte Dicke von maximal 0,15 mm auf. Die Gesamtlänge eines Segmentes (einschließlich der sich verjüngenden Fläche bis zur Verbindung mit dem Katheterschaft) beträgt bevorzugt bis zu 280 mm. So kann zu Beginn einer Ablationsprozedur der Katheter nasal oder oral in Richtung Magen vorgeschoben werden, wobei dessen distaler Teil den Mageneingang erreicht. Aufgrund der vorzugsweisen Länge von 240 mm des Messbereichs kann der gesamte relevante Teil des Ösophagus erfasst werden.

Da der Durchmesser eines Ösophagus unterschiedlich ausfällt, ist mit einer bevorzugten erfindungsgemäßen Temperatursonde bei minimalem anzunehmendem Ösophagusdurchmesser von 15 mm und einem maximal anzunehmendem Ösophagusdurchmesser von 35 mm die Abdeckung der gesamten zu beobachtenden Oberfläche des Ösophagus gegeben.

Vorzugsweise sind die einzelnen Sensor-Array-Segmente so um den Katheter angeordnet, dass sie axial teilweise überlappend um den Katheter liegen und mit dem Schaft des Katheters an dessen distalem Ende über eine Verbindungsstelle formschlüssig verbunden sind. Die sich verjüngenden Anschlussbereiche der Sensor-Array-Segmente sind am distalen Ende z.B. durch Aussparungen ins Innere des Katheters geführt. Zwischen Schaft des Katheters und Verbindungsstelle liegen sie vorzugsweise eingepresst vor. Die Leiterbahnen, die die Sensoren verbinden, führen in das Lumen des Katheters und sind dort an Überführungsleiter angeschlossen, die zur Auswerte- und Steuereinheit führen.

Die Sensor-Array-Segmente sind bevorzugt mehrschichtig aufgebaut. Zwischen zwei Trägerschichten, die als Isolator fungieren, d.h. einer Kunststoffschicht, die elektrisch isolierend wirkt, befindet sich eine Kopplungsschicht mit eingebetteten Temperatursensoren und Leiterbahnen. Die jeweiligen Schichten sind bevorzugt zwischen 0,01 und 0,05 mm dick. Die Trägerschichten sind z.B. aus Kapton oder Polyamid 12. Die Kopplungsschicht mit der eingebetteten Elektronik ist z.B. aus Polydimethylsiloxan (PDMS). Die Kunststoffschicht des Trägers verhindert zum einen unkontrollierten Fluss des Messstroms zwischen den Sensoren aufgrund der feuchten, leitfähigen Umgebung und somit eine Verfälschung des Messergebnisses und zum anderen wird ein Stromfluss zwischen den Sensoren und der umgebenden Schleimhaut minimiert, welcher durch eine mögliche Induktion von Strom in das Sensornetz, z.B. in Folge von RF-Ablation hervorgerufen wird. Solche Ströme könnten zu einer Schädigung der Ösophagusoberfläche führen. Das Risiko von Gewebeschäden aufgrund von induzierten Strömen in das Sensornetz durch RF-Ablation kann somit minimiert werden.

Die Herstellung solcher flächigen Segmente ist dem Fachmann bekannt, z.B. Rogers, John A.; Ghaffari, Rootbeh; Kim, Dae-Hyeong: "Strechable Bioelectronics for Medical Devices and Systems"; Springer; 2016.

Die Elektronik inklusive der Temperatursensoren, der Leiterbahnen (bevorzugt aus einer Silberlegierung oder Graphen) und der Kontaktierungen auf den Verbindungsstellen werden vorzugsweise durch additive Verfahren, wie Aerosoldruck oder Druck mit leitfähigen Tinten, gefertigt.

Die röntgensichtbaren Marker werden bevorzugt durch additive Prozesse, wie Aerosoldruck, auf die flächigen Sensor-Array-Segmente und/oder den Katheterschaft aufgebracht. Der Marker kann im Bereich des Katheterschafts eine ringförmige und im Bereich der Segmente eine balkenartige Form aufweisen, welche sich zumindest über einen Teil des Sensor-Array-Segmentes oder des Schafts erstreckt. Die Balken besitzen vorzugsweise eine Breite von 1 bis 3 mm. Die Marker sind bevorzugt aus Tantal oder einer Titanlegierung, wie Ti 6AL7NB (TAN).

Weiterhin bevorzugt weist der Katheter am distalen Ende eine atraumatische Spitze auf, vorzugsweise aus Silikon, die ggf. mit der davor liegenden Verbindungsstelle für die Enden der Sensor-Array-Segmente eine Einheit bildet und formschlüssig mit dem Katheter endet. Die Leiterbahnen, die die Sensoren verbinden, können Dehnungszonen aufweisen, indem sie z.B. mäanderförmig, oder in Form von Serpentinen angeordnet sind.

Eine bevorzugte erfindungsgemäße Temperatursonde besitzt mit anliegenden gefalteten Sensor-Array-Segmenten auf dem Katheter maximal einen Durchmesser von 4,5 mm, wobei der Katheter einen maximalen Durchmesser von 2,0 mm aufweist.
In der ersten bevorzugten Ausführungsvariante der Erfindung weisen die einzelnen Segmente eine sich verjüngende Fläche zum distalen Ende des Katheterschaftes auf. D.h. die Verjüngung der Segmentfläche erfolgt in Richtung der Verbindungsstelle von Segmentmaterial und Katheterschaft. In einer Ausführungsform ist jedes Sensor-Array-Segment am distalen Ende mit dem Katheterschaft verbunden. Im Bereich der Verbindungsstelle zwischen den flächigen Sensor-Array-Segmenten und dem Katheterschaft kann das Material eine, im Vergleich zum restlichen Segmentmaterial, Federwirkung besitzen, die erzeugt werden kann, indem man das Material unterschiedlich bearbeitet. So wird die graduelle Federwirkung der Sensor-Array-Segmente eingestellt, damit sich das Segment in Richtung Ösophagusoberfläche wölbt, indem vorzugsweise die Eigenschaften des Materials lokal, selektiv manipuliert werden. Das kann z.B. durch eine einseitige Wärmebehandlung des mehrschichtigen Trägermaterials erfolgen, was zu einer unterschiedlichen Spannung in den einzelnen miteinander verbundenen Schichten und somit zu einer Biegung führt. Da die resultierende Federkraft gering gehalten wird, kann sie keine Verschiebung oder Aufweitung des Ösophagus hervorrufen.

Zum effektiven Einführen der Temperatursonde in den Ösophagus können die flexiblen Sensor-Array-Segmente am Schaft des Katheters mit einer axial auf dem Katheter beweglichen Einführhülse verbunden sein. Diese bewegliche Einführhülse fixiert die Sensor-Segmente überlappend gefaltet am Katheterschaft. Sie ist ebenfalls aus biegsamem Material, wie thermoplastischen Elastomeren, thermoplastischem Polyesterelastomeren, thermoplastischen Elastomeren auf Olefinbasis und weist einen der Temperatursonde angepassten Durchmesser auf. Ihre Länge entspricht bevorzugt ca. 350 mm, mindestens jedoch 280 mm.

Die erzeugte Federwirkung bewirkt nach Einführen in den Ösophagus und nach Abziehen der Einführhülse, dass die Segmente vom Schaft weggedrückt werden und begünstigt so einen Kontakt zwischen Schleimhaut und flexiblen Sensor-Segmenten mit den integrierten Temperatursensoren. Nach Entfernen der Einführhülse öffnen sich die Sensor-Array-Segmente quasi in Form eines Regenschirms und liegen so durch Adhäsion an der Ösophagusoberfläche an.

Zum verbesserten Einführen kann die Temperatursonde im Inneren des Katheters einen Versteifungsdraht umfassen, wobei der Katheter am distalen Ende geschlossen ist. Um eine Perforation des Versteifungsdrahtes am distalen Ende zu verhindern, reicht dieser maximal bis zum distalen Endes des Katheters.

Durch die Vielzahl an Sensoren, in bevorzugter Weise 180 Sensoren gleichmäßig auf sechs Sensor-Array-Segmente verteilt, ist eine Temperaturmessung mit hoher räumlicher Auflösung ohne Sensorschatten über Kontakt möglich.

In einer zweiten bevorzugten Ausführungsform ist jedes Sensor-Array-Segment sowohl am distalen Ende mit dem Katheterschaft wie oben beschrieben, als auch am proximalen Ende mit einer in Längsrichtung zur Katheterspitze auf dem Katheter beweglichen Hülse verbunden. Die einzelnen Segmente weisen für diese Variante neben der sich verjüngenden Fläche in distaler Richtung zur Verbindungsstelle von Sensor-Segment und Katheterschaft, eine weitere sich verjüngende Fläche in proximaler Richtung zu einer Verbindungsstelle zur beweglichen Hülse auf. Am Beginn der verjüngenden Seite sind die jeweiligen röntgendichten Markerbereiche angeordnet. Gegebenenfalls befindet sich eine Begrenzung des Vorschubs für die Hülse auf dem Katheterschaft am distalen Ende in Richtung zur Katheterspitze. Nach dem Einführen der Temperatursonde in den Ösophagus wird die bewegliche Hülse in Richtung Katheterspitze verschoben, so dass die Sensor-Segmente sich quasi als Segel entfalten können und mittels Adhäsion an der Oberfläche des Ösophagus haften.

Die erfindungsgemäße Temperatursonde weist in einer dritten bevorzugten Ausführungsform, in Ergänzung zur ersten und zweiten bevorzugten Ausführungsform, einen entfaltbaren Ballon auf, der sich zwischen den flächigen Sensor-Segmenten und dem Katheterschaft angeordnet beweglich befindet. Dadurch lässt sich die Temperatursonde im Bereich des flexiblen Trägermaterials auch mittels Ballon entfalten, so dass sich das Trägermaterial mit den Sensoren möglichst nah an die Oberfläche des Ösophagus anlegt. Der Ballon wird dazu nach dem Einführen der Temperatursonde in den Ösophagus zur Entfaltung bevorzugt mit einem Gas, z.B. Raumluft, befüllt. Im Anschluss an das Anlegen der Sensoren auf den Sensor-Array-Segmenten an die Ösophaguswand wird der Ballon entlüftet und die flächigen Segmente mit den Sensoren bleiben aufgrund von Van-der-Waals-Kräften zwischen Segmenten und feuchter Schleimhaut an der Oberfläche des Ösophagus haften. So wird auch mit dieser Lösung keine Kraft während einer Ablation auf die Oberfläche des Ösophagus ausgeübt.

Die flexiblen Sensor-Array-Segmente sind dabei einseitig, distal vom Ballon mit dem Katheterschaft, oder zweiseitig, distal vom Ballon mit dem Katheterschaft und proximal vom Ballon mit einer auf dem Katheterschaft in Längsrichtung zur Katheterspitze beweglichen Hülse, verbunden, so dass es keine fixierte Verbindung zwischen Ballon und Sensor-Array-Segmenten gibt. Alle drei Ausführungsvarianten haben eine Hülse gleicher Beschaffenheit.

Die Befüllung des Ballons wird über die Steuereinheit des Katheters überwacht, so dass jederzeit ersichtlich ist, ob und wie weit der Ballon gefüllt ist, so dass eine mögliche Ablation solange unterbrochen werden kann, entweder automatisch oder durch Feedback an den Anwender, bis eine vollständige Entlüftung des Ballons stattgefunden hat. Auf diese Weise wird eine ungewollte Kraftwirkung auf die Ösophagusoberfläche ausgeschlossen. In bevorzugter Weise wird der Ballon nur einmal im Vorfeld der Ablation befüllt und unmittelbar vor der ersten Ablation vollständig entlüftet. Die Temperatursensoren messen aufgrund ihres Kontakts zur Ösophagusoberfläche die Temperaturverteilung auf eben dieser und nicht die Temperaturveränderung im Lumen. Die gewonnenen Informationen über die Temperaturverteilung der Ösophagusoberfläche ermöglichen ein Feedback gegenüber Anwender oder Steuereinheit.

Die oben beschriebenen Temperatursonden sind bevorzugt Teil eines Kathetersystems mit einem Katheter zur Einführung der Temperatursonde in den Ösophagus, einer Auswerteeinheit, zur Verarbeitung der von den Temperatursensoren gelieferten Messwerten, und einer Steuereinheit zur Weitergabe von Informationen an den Anwender und besonders bevorzugt an das Ablationssystem, zur Verringerung oder Aussetzung der Leistungsabgabe des Ablationssystems. Bevorzugt umfasst die Vorrichtung auch eine Warneinrichtung zur akustischen, visuellen oder taktilen Wiedergabe von Temperaturinformationen, insbesondere von Über- und Unterschreitungen des akzeptablen Temperaturbereiches. In einer bevorzugten Ausführung erfolgt über die ermittelten Temperaturwerte der Temperatursonde eine direkte Rückkopplung an die Ablationsgeräte, indem bei Über- oder Unterschreiten der Grenzwerte die Ablationsenergie verringert oder ausgeschaltet wird.

### Ausführungsbeispiel:

Die Erfindung wird nun anhand von Ausführungsbeispielen mit Bezug auf die beiliegenden Zeichnungen näher erläutert.

### Legende zu den Abbildungen

1 - Herz
2 - Ösophagus (Speiseröhre)
3 - Katheter
4 - Temperatursonde
5 - Schnittstelle zur Steuereinheit
6 - Nasen-Rachen-Raum
7 - Stelle am linken Herzvorhof an dem die Spitze des Ablationskatheters liegt
8 - linker Herzvorhof
9 - Temperatursensor
10 - röntgendichter Markerbereich (vzw. Balken aus Tantal)
11 - atraumatische Spitze Katheter (vzw. aus Silikon)
12 - Mageneingang
13 - unterer Ösophagusbereich
14 - Sensor-Array-Segmente der Sonde (mehrschichtig - 19 und 20)
15 - sich verjüngende Anschlussbereiche der Segmente
16 - Verbindungsstelle am distalen Ende des Katheters
17 - Leiterbahnen (Ag-Legierung oder Graphen)
18 - Überführungsleiter zur Auswerte- und Steuereinrichtung
19 - Kopplungsschicht mit eingebetteter Elektronik (9, 17) (vzw. aus PDMS)
20 - Trägerschicht (vzw. aus Kapton oder Polyamid 12)
21 - (spaltfüllender) Kleber (vzw. Cyanacrylat Klebstoff 4902 Fa. Henkel)
22 - Aussparungen
23 - Lumen des Katheters 3
24 - Logikmodul (Multiplexer + Operationsverstärker)
25 - Dehnungszonen in Leiterbahnen 17
26 - Einführhülse
27 - Verbindungsstelle am proximalen Ende des Katheters
28 - bewegliche Hülse zum distalen Ende
29 - Ballon

In den Zeichnungen zeigen:
Fig. 1 die anatomische Lage von Herz 1 und Ösophagus 2, sowie die schematische Darstellung einer Temperatursonde 4 innerhalb des Ösophagus 2;
Fig. 2 eine beispielhafte Ausführungsform eines Katheters 3 mit Temperatursonde 4 die im Ösophagus 2 platziert ist und mit dessen Messbereich auf den herznahen Bereich ausgerichtet ist;
Fig. 3 eine vergrößerte Darstellung einer ersten bevorzugten Ausführungsform der erfindungsgemäßen Temperatursonde 4 eines Katheters zur Temperaturmessung im Ösophagus 2;
Fig. 4 den schematischen Aufbau eines Sensor-Array-Segments 14 als Querschnitt;
Fig. 5 die Darstellung der Verbindungsstelle 16 eines Sensor-Array-Segments 14 mit dem Schaft des Katheters;
Fig. 6 die Verbindung von Leiterbahnen 17 des Sensor-Array-Segments 14 mit Überführungsleitern;
Fig. 7 die schematische Darstellung der Temperatursensoren 9 und der Leiterbahnen 17 des Sensor-Array-Segments 14;
Fig. 8 die schematische Darstellung der Einführhülse 26 und der Faltung der Sensor-Array-Segmente 14;
Fig. 9 eine vergrößerte Darstellung einer zweiten bevorzugten Ausführungsform der erfindungsgemäßen Temperatursonde 4 eines Katheters zur Temperaturmessung im Ösophagus 2 mit Befestigung der Sensor-Array-Segmente 14 an einer auf dem Katheterschaft beweglichen Hülse;
Fig. 10 die Darstellung einer dritten bevorzugten Ausführungsform der erfindungsgemäßen Temperatursonde 4 eines Katheters zur Temperaturmessung im Ösophagus 2 mit Ballon 29 als Modifizierung der zweiten bevorzugten Ausführungsform;
Fig. 11 Querschnitte durch den Ballon 29 und die Sensor-Array-Segmente 14 bei unterschiedlichen Befüllungsstadien des Ballons 29.

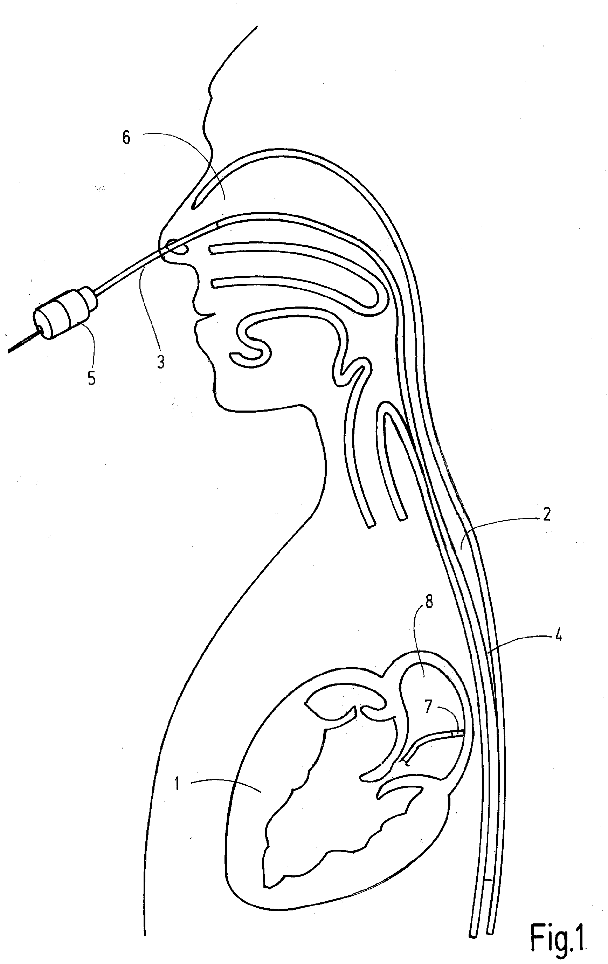

Fig. 1 zeigt schematisch die Lage von Herz 1 und Ösophagus 2. In den Ösophagus 2 ist ein Katheter 3 mit einer Temperatursonde 4 und Schnittstelle 5 zur Steuereinheit (nicht dargestellt), vorzugsweise über den Nasen-Rachen-Raum 6, eingeführt. Der Ösophagus 2 liegt anatomisch dicht am linken Vorhof 8, in welchem die Spitze des Ablationskatheters 7 dargestellt ist.

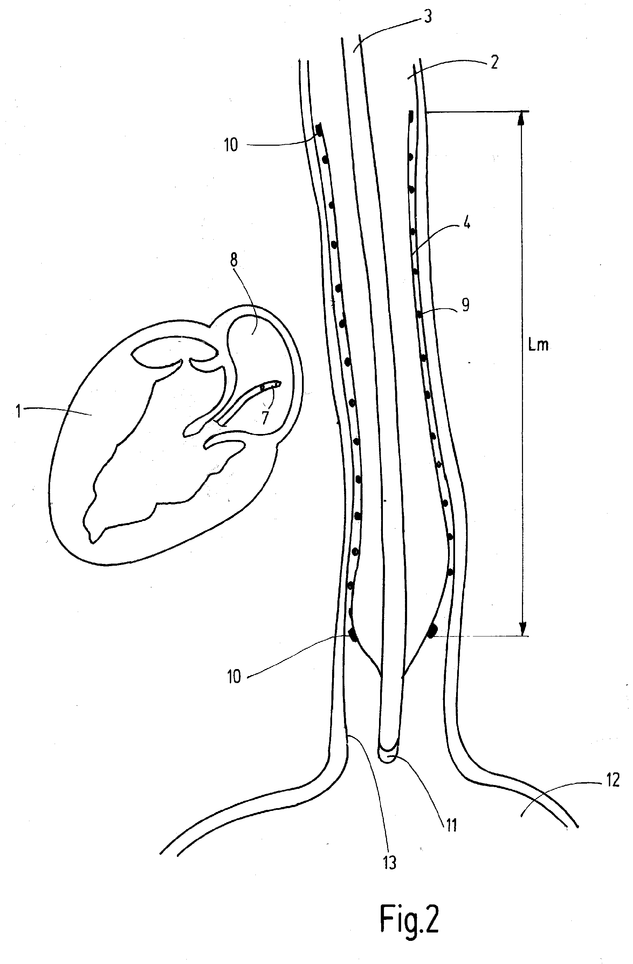

In Fig. 2 ist eine beispielhafte Ausführungsform eines Katheters 3 mit Temperatursonde 4 im Querschnitt dargestellt, wobei die Temperatursensoren 9 Kontakt zur Ösophagusoberfläche haben. Der Katheter 3 mit der Temperatursonde 4 wird bis in den unteren Teil des Ösophagus 13 vorgeschoben und weist zum Schutz vor Gewebebeschädigung an seinem distalen Ende eine atraumatische Spitze 11 auf.
Mit Erreichen des unteren Ösophagusbereichs 13 deckt die Temperatursonde 4 im gestreckten Zustand den gesamten relevanten Messbereich ab. Zur Visualisierung der Lage der Temperatursonde 4 mit Hilfe von Röntgenaufnahmen ist der Messbereich der Temperatursonde 4 durch röntgendichte Marker 10, welche sich am proximalen und distalen Ende der Temperatursonde 4 befinden und vorzugsweise aus Tantal bestehen, abgegrenzt. Im Bereich zwischen den röntgendichten Markern 10 befindet sich der Messbereich Iₘ der Temperatursonde 4 mit einer Vielzahl an Temperatursensoren 9 mit einer bevorzugten Länge zwischen 200 und 250 mm. Aufgrund der Länge des Messbereichs Iₘ der Temperatursonde 4 ist eine aufwendige Ausrichtung des Messbereichs entlang der Längsachse des Ösophagus 2, wie sie bei derzeit bekannten Temperatursonden für den Ösophagus nötig ist, auf den dem Ablationsort nahe liegenden, relevanten zu untersuchenden Bereich der Ösophagusoberfläche nicht nötig. Der Ösophagus weist im Normalfall eine Länge von 250-280 mm auf, dementsprechend kann mit dem bevorzugten Messbereich der Temperatursonde 4, nimmt man die distalen und proximalen Bereiche des Ösophagus 2 aus, die gesamte relevante Länge des Ösophagus 2 erfasst werden. Ebenfalls ist aufgrund der Länge eine ausreichende Sicherheit gegen eine Verkürzung durch Verdrehung und Stauchung des Messbereiches der Temperatursonde 4 gegeben.

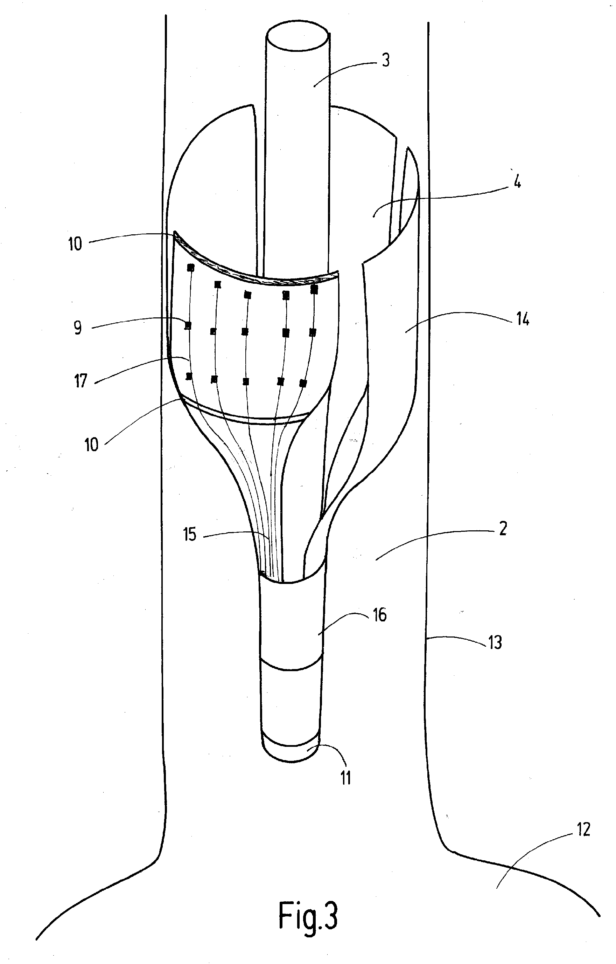

Fig. 3 zeigt die schematische, vergrößerte Darstellung einer ersten bevorzugten Ausführungsform einer Temperatursonde 4 eines Katheters 3 zur Temperaturmessung im Ösophagus 2. Die Temperatursonde 4 besteht aus mehreren flexiblen Sensor-Array-Segmenten 14, wobei die einzelnen Segmente 14 eine sich verjüngende Fläche 15 in Richtung Katheterspitze, beginnend nach dem distalen Marker 10 aufweisen, deren Verjüngung in Richtung der Verbindungsstelle 16 von Sensor-Array-Segmenten 14 und Katheter 3 erfolgt. In einer bevorzugten Ausführungsform ist jedes Segment 14 am distalen Ende des Katheters 3 mit diesem verbunden.
Am distalen Ende des Katheters 3 befindet sich eine atraumatische Spitze 11, bevorzugt aus Silikon, welche beim Bewegen des Katheters 3, zum Beispiel beim Einführen über den nasalen Zugang, verhindert, dass umliegendes Gewebe wie beispielsweise die Ösophaguswand beschädigt wird. Die atraumatische Spitze 11 weist eine sphärische Kontur auf und ist entlang der Längsrichtung des Katheters 3 durch geringe Krafteinwirkung stauchbar. Vorzugsweise wird die sphärische Kontur der atraumatischen Spitze 11 durch eine dünne Silikonmembran über einem Hohlraum gebildet, wodurch eine hohe Flexibilität gegeben ist. Die atraumatische Spitze 11 ist bevorzugt über einen Formschluss mit dem Katheter 3 verbunden.
Der flexible Schaft des Katheters 3 mit einem bevorzugten Außendurchmesser von 2 mm weist im Inneren einen Kanal zur Einführung eines Versteifungsdrahtes auf, welcher den Katheter 3 distal nicht verlassen kann. In Abhängigkeit der eingeführten Länge des Versteifungsdrahts kann die Flexibilität des Katheters 3 manipuliert werden.
Nachdem die Sonde nasal oder oral in den Ösophagus 2 des Patienten eingeführt und bis zum unteren Bereich des Ösophagus 13 vorgeschoben wurde, legen sich die Sensor-Array-Segmente 14 mit der Vielzahl an Temperatursensoren 9 an der Ösophagusoberfläche an, was durch eine leichte Federwirkung der Sensor-Array-Segmente 14, im Bereich der Verbindungsstelle 16 unterstützt wird.
Die einzelnen Sensor-Array-Segmente 14 mit einer bevorzugten Länge von 240 mm und einer bevorzugten Breite von 21 mm weisen eine bevorzugte Anzahl von 30 Temperatursensoren 9 auf, die durch elektrisch leitfähige Bahnen 17 mit einer Auswerteeinheit (nicht dargestellt) verbunden sind. In einer weiteren bevorzugten Ausführungsform können mehrere Temperatursensoren 9 über elektrisch leitfähige Bahnen 17 miteinander verbunden sein.
Die elektrisch leitfähigen Bahnen 17 und Temperatursensoren 9 bestehen bevorzugt aus einer Silberlegierung oder Graphen. Der Bereich der Temperatursensoren 9 auf den Sensor-Array-Segmenten 14 wird durch balkenförmig ausgeprägte röntgendichte Marker 10 begrenzt.

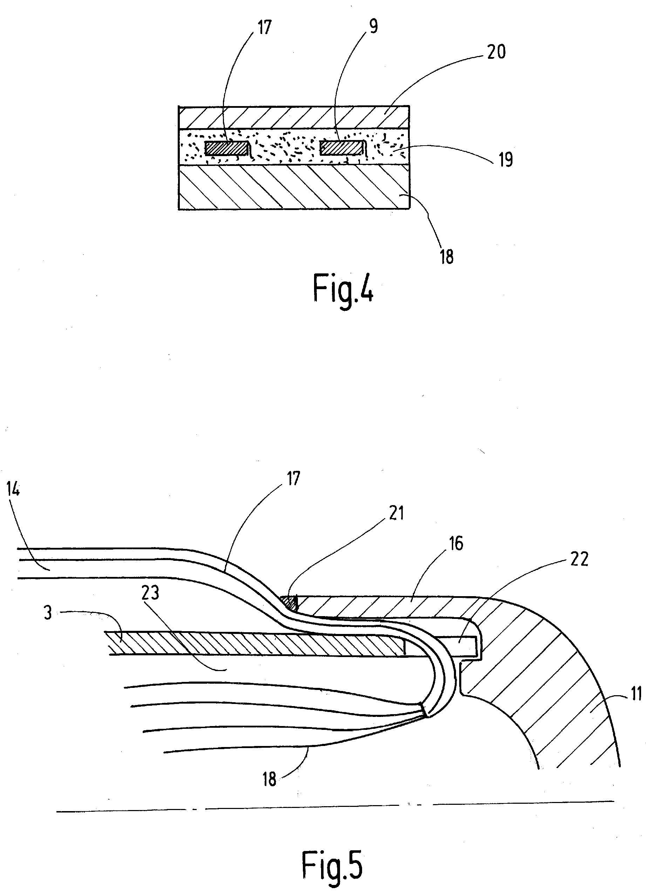

In Fig. 4 ist der schematische Aufbau eines Sensor-Array-Segments 14 als Querschnitt dargestellt. Die Sensor-Array-Segmente 14 sind hoch flexibel und dünn, um mit dem Katheter 3 als Trägersystem durch den nasalen oder oralen Zugang in den Ösophagus 2 eingeführt und an der Schleimhaut angelegt werden zu können, wobei die Sensor-Array-Segmente 14 Änderungen der Oberflächenkontur nachvollziehen.
Als Trägermaterial 20 für die Sensor-Array-Segmente 14 wird vorzugsweise Kapton oder Polyamid 12 mit einer Dicke von maximal 0,05 mm eingesetzt. Das Trägermaterial 20 stellt die beiden äußeren Schichten des mehrschichtig aufgebauten Sensor-Array-Segments 14 dar, welches als elektrischer Isolator wirkt, um zu verhindern, dass es zu einem Stromfluss zwischen Sensor-Array-Segment 14 und Ösophagusoberfläche z.B. durch Induktion, aufgrund von RF-Ablation, in die elektrischen Leiter und Sensoren kommt.
Zwischen den Schichten des Trägermaterials 20 befindet sich eine Kopplungsschicht 19, welche die eingebettete Elektronik 9, 17 mit dem Trägermaterial 20 verbindet. Die Kopplungsschicht 19 besteht vorzugsweise aus Polydimethylsiloxan (PDMS) und weist vorzugsweise eine Dicke von weniger als 0,05 mm auf. Innerhalb der Kopplungsschicht 19 sind die elektrisch leitfähigen Strukturen 17 und die Temperatursensoren 9 eingebettet. Die elektrisch leitfähigen Strukturen 17 und die Temperatursensoren 9 bestehen vorzugsweise aus Silberlegierungen oder Graphen.
Aufgrund der unterschiedlichen Materialien, insbesondere der unterschiedlichen Elastizitätsmodule von Trägermaterial 20 und Elektronik 9, 17 kommt es bei der Biegung des Sensor-Array-Segments 14 zu unterschiedlich stark ausgeprägten Dehnungen in den Materialschichten. Die Kopplungsschicht 19 verhindert ein Ablösen und Aufbrechen der eingebetteten Elektronik, indem es die Materialdehnungen voneinander trennt. Der geschichtete Aufbau der Sensor-Array-Segmente 14 ist im Bereich der röntgendichten Marker 10 um eine weitere Schicht ergänzt, diese fungiert als elektrischer Isolator zwischen röntgendichtem Marker 10 und eingebetteter Elektronik 9, 17, insbesondere im Bereich von Kreuzungen dieser Elemente.

Fig. 5 zeigt die Schnittdarstellung der Verbindungsstelle 16 mit atraumatischer Spitze 11, wobei in der dargestellten bevorzugten Ausführungsform Verbindungselement 16 und atraumatische Spitze 11 als zusammenhängendes Bauteil ausgeführt sind. Atraumatische Spitze 11 und Verbindungselement 16 sind formschlüssig mit dem Schaft des Katheters 3 verbunden, wobei die Anschlussbereiche der Sensor-Array-Segmente 14 zwischen Schaft des Katheters 3 und Verbindungelement 16 eingepresst sind. Die Verbindungsbereiche der Sensor-Array-Segmente 14 werden durch Aussparungen 22 ins Innere des Katheters 3 geführt. Spalten zwischen dem Schaft des Katheters 3, Sensor-Array-Segmenten 14 und Verbindungselement 16 sind vorzugsweise durch einen spaltfüllenden Kleber 21, wie dem Cyanacrylat-Klebstoff 4902 der Firma Henkel, gefüllt. Die einzelnen leitfähigen Strukturen 17 zur Konnektierung der Temperatursensoren 9 werden im sich verjüngenden Sensor-Array-Segment 14 in das Lumen 23 des Katheters 3 geführt, wo sie an Überführungsleiter 18 angeschlossen sind.

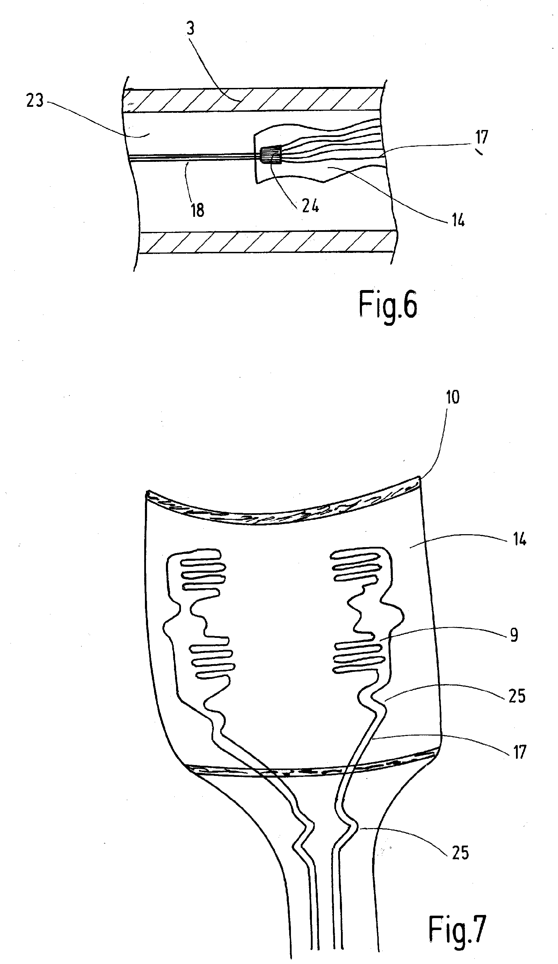

Fig. 6 zeigt die Verbindung von Leiterbahnen 17 des Sensor-Array-Segments 14 mit den Überführungsleitern 18 innerhalb des Lumen 23 des Katheters 3. Über ein Logikmodul 24, vorzugsweise bestehend aus Multiplexer und Operationsverstärker, wird die Anzahl der benötigten Überführungsleiter, welche zur Schnittstelle 5 und zur Steuereinheit geführt werden, reduziert.

In Fig. 7 ist die schematische Darstellung der Temperatursensoren 9 und der Leiterbahnen 17 des Sensor-Array-Segments 14 dargestellt. Die Leiterbahnen 17 weisen Dehnungszonen 25 auf, um eine ausreichende Dehnung bei akzeptabler Materialspannung zu gewährleisten, ohne dass es zu einem Versagen der Elektronik 9, 17, zum Beispiel durch Bruch, kommt.
Die Temperatursensoren 9 sind vorzugsweise mäanderförmig aufgebaut und umfassen jeweils eine Fläche von vorzugsweise 20 mm x 6 mm. Besonders bevorzugt sind auf einem Sensor-Array-Segment 14 jeweils zehn Temperatursensoren 9 in Längs- und je drei in Querrichtung angeordnet, wobei die Sensor-Array-Segmente 14 vorzugsweise eine Länge von 240 mm und eine Breite von 21 mm haben. In der bevorzugten Ausführungsform sind 6 Sensor-Array-Segmente 14 rotationssymmetrisch um den Schaft des Katheters 3 angeordnet.

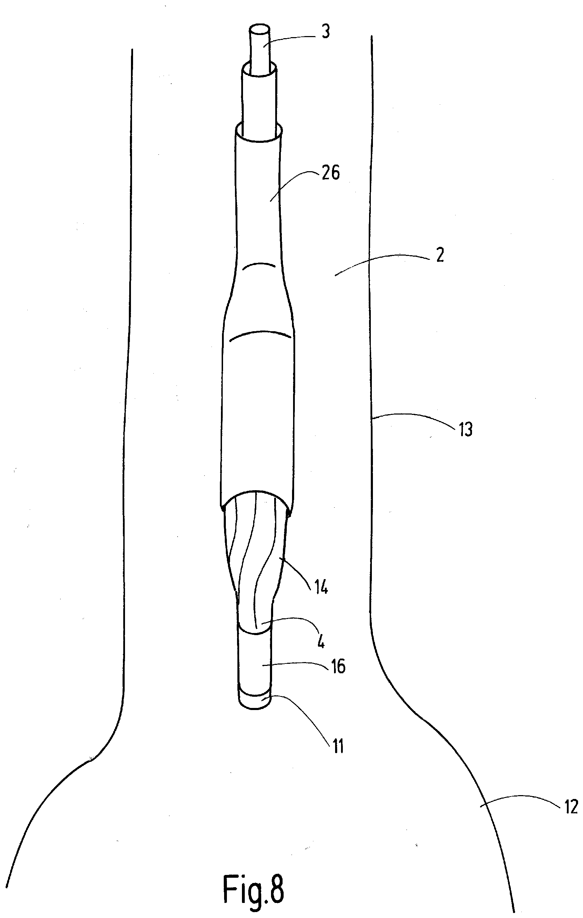

In Fig. 8 ist die Einführhülse 26 und die Faltung der Sensor-Array-Segmente 14 schematisch dargestellt. Um eine Wölbung der Sensor-Array-Segmente 14 beim Einführen in den Körper zu verhindern, sind die angelegten Sensor-Array-Segmente 14 durch eine dünne Einführhülse 26 am Schaft fixiert, welche zurückgezogen wird, sobald sich die Temperatursonde 4 im Ösophagus 2 befindet. In der bevorzugten Ausführungsform weist die Temperatursonde 4, mit anliegenden Sensor-Array-Segmenten 14 innerhalb der Einführhülse 26, einen maximalen Durchmesser von 4,5 mm auf.
Die Sensor-Array-Segmente 14 können durch Überlappung am Schaft des Katheters 3 angelegt werden, wie es etwa von der Faltung von Regenschirmen bekannt ist. Die Sensor-Array-Segmente 14 können, aufgrund ihrer sich zur Verbindungsstelle 16 hin verjüngenden Form, im gewölbten Zustand einen Durchmesser des Ösophagus 2 von bis zu 35 mm lückenlos abdecken, wobei es zu einer teilweisen Überlappung der Sensor-Array-Segmente 14 in Abhängigkeit vom Durchmesser kommt.
Beim Entfernen des Katheters 3 zur Temperaturmessung im Ösophagus 2 klappen die Sensor-Array-Segmente 14 sich entgegen der Zugrichtung des Katheters 3 um und werden hinterhergezogen, wobei es, aufgrund der Flexibilität und Weichheit der Materialien, zu keiner Schleimhautverletzung beim Patienten kommt.

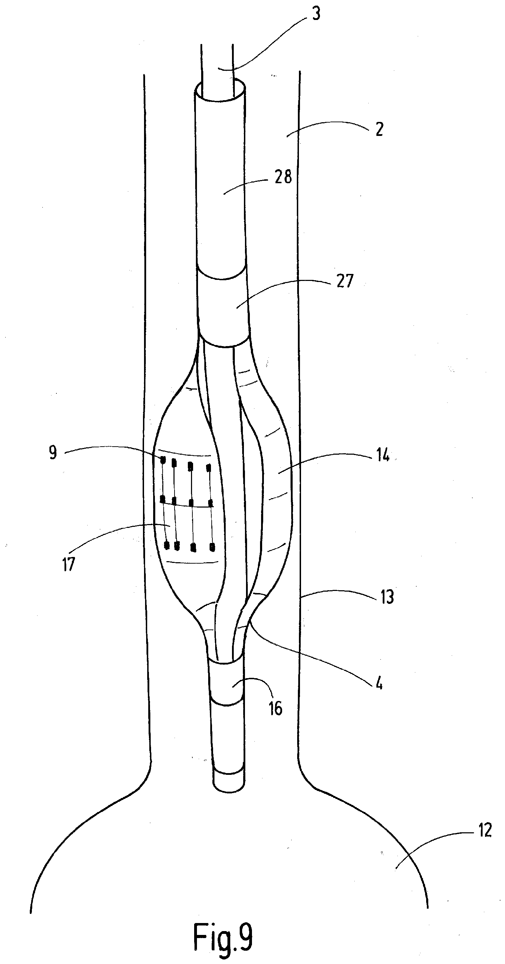

Fig. 9 zeigt eine zweite bevorzugte Ausführungsform der erfindungsgemäßen Temperatursonde 4 eines Katheters zur Temperaturmessung im Ösophagus 2. Dabei ist die erste bevorzugte Ausführungsform in der Art modifiziert, dass die Sensor-Array-Segmente 14 sich distal und proximal in Längsrichtung des Schafts des Katheters 3 verjüngen. Die proximale Verjüngung des Sensor-Array-Segments 14 ist mit einer auf dem Schaft des Katheters 3 in Längsrichtung beweglichen Hülse 28 über eine Verbindungsstelle 27 verbunden.
Nach dem Einführen in den Ösophagus 2 wird die bewegliche Hülse durch den Anwender in Richtung Katheterspitze geschoben, wodurch sich die Sensor-Array-Segmente 14 segelartig aufstellen und gegen die Ösophagusoberfläche 13 gedrückt werden und dort aufgrund der Adhäsion haften bleiben. Nachdem die Sensor-Array-Segmente 14 an der Ösophagusoberfläche anliegen, muss der Anwender die bewegliche Hülse nicht weiter verschieben oder fixieren.
Um nur eine definierte Aufwölbung der Sensor-Array-Segmente 14 zu erlauben, ist eine Begrenzung des Vorschubs der beweglichen Hülle 28 vorgesehen. Nach dem Ende der Ablation kann die bewegliche Hülse 28 proximal entlang des Schaftes des Katheters 3 bewegt werden, um die Sensor-Array-Segmente 14 an den Katheter 3 anzulegen. Aufgrund der Flexibilität und Weichheit der Materialien der Sensor-Array-Segmente 14 kommt es zu keiner Verletzung oder Blockade innerhalb des Patienten.

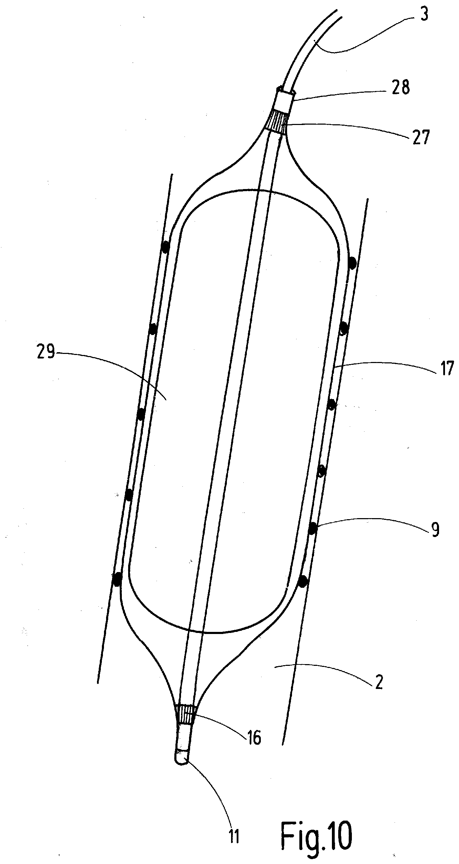

Fig. 10 zeigt im Schnitt eine dritte bevorzugte Ausführungsform der erfindungsgemäßen Temperatursonde 4 eines Katheters 3 zur Temperaturmessung im Ösophagus 2. Dabei ist die erste oder zweite bevorzugte Ausführungsform in der Art modifiziert, dass sich zwischen Sensor-Array-Segmenten 14 und Schaft ein Ballon 29 befindet, wie er von Ballonkathetern bekannt ist. Dieser positioniert die Sensor-Array-Segmente 14 nach Befüllung durch den Anwender an der Ösophagusoberfläche 13. Vor der Ablation wird der Ballon 29 entlüftet und die Sensor-Array-Segmente 14 bleiben aufgrund der van-der-Waals-Kräfte an der Ösophagusoberfläche haften, so dass während der Ablation keine Kräfte auf den Ösophagus 2 ausgeübt werden.
Die vorzugsweise Befüllung des Ballons mit Raumluft erfolgt von Hand, beispielsweise mit Hilfe einer Spritze und der Fülldruck wird durch eine Steuereinheit überwacht, die im Falle einer Grenzwertüberschreitung eine Entlüftung des Ballons 29 vornimmt und/oder dem Anwender taktile, visuelle oder akustische Warnmeldungen gibt.

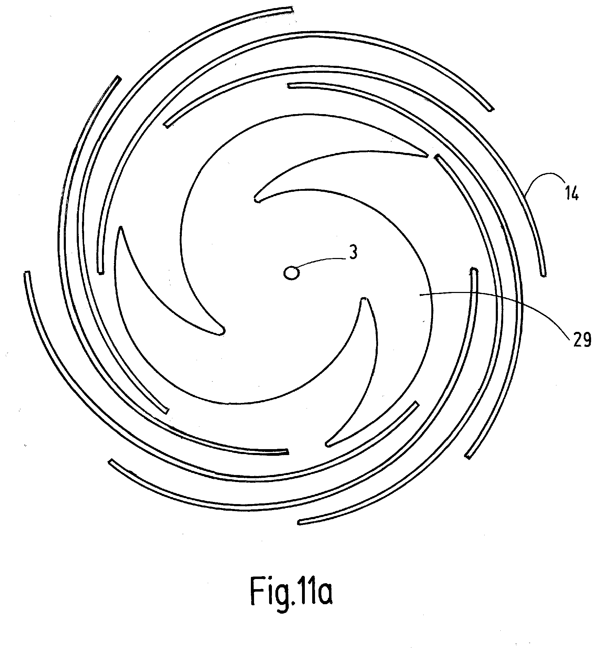

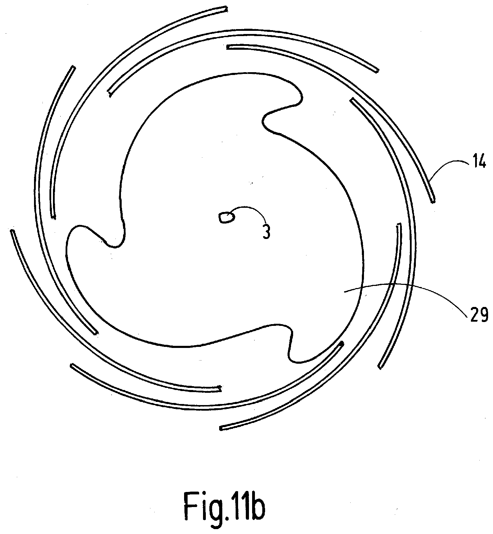

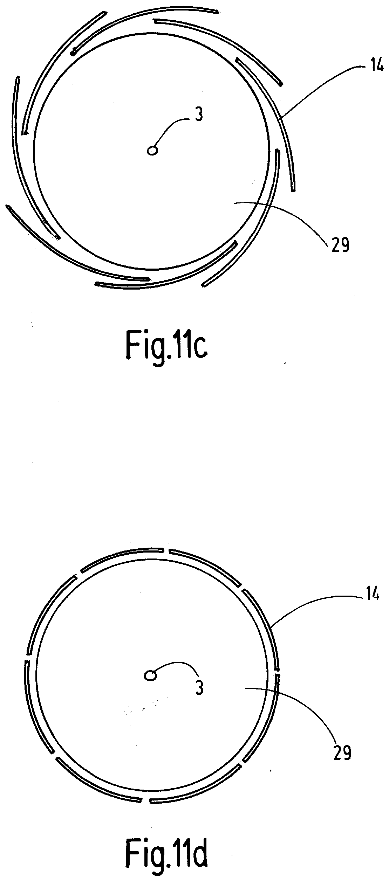

Fig. 11a bis 11d zeigen an Hand der dritten bevorzugten Ausführungsform die Überlappung der Sensor-Array-Segmente 14 bei verschiedenen Befüllungsstadien des Ballons 29.

## Patentansprüche

1. Temperatursonde (4) zur Einführung in den Ösophagus (2), **dadurch gekennzeichnet, dass** sie mindestens zwei flexible flächige Sensor-Array-Segmente (14) aufweist, die um einen Katheter (3) rotationssymmetrisch angeordnet sind und deren Flächen sich mindestens zum distalen Ende des Katheters (3) verjüngen, so dass diese Anschlussbereiche (15) bilden, welche am distalen Ende mit dem Katheter (3) verbunden sind, wobei die Sensor-Array-Segmente (14) jeweils eine oder mehrere Temperatursensoren (9) aufweisen, welche über Leiterbahnen (17) verbunden sind, wobei die Temperatursensoren (9) mit der Schleimhautoberfläche des Ösophagus (2) funktional kontaktierbar sind, so dass über die Temperatursensoren (9) eine Temperatur an der Schleimhautoberfläche erfassbar ist.

2. Temperatursonde (4) nach Anspruch 1, **dadurch gekennzeichnet, dass** die Sensor-Array-Segmente (14) jeweils distal und proximal röntgendichte Markerbereiche aufweisen, zwischen denen die Temperatursensoren (9) angeordnet sind, die von Marker zu Marker einen Messbereich Iₘ definieren.

3. Temperatursonde (4) nach Anspruch 1 oder 2, **dadurch gekennzeichnet, dass** die Temperatursensoren (9) in den Sensor-Array-Segmenten (14) vom proximalen zum distalen Marker mäandernd oder in Reihe(n) aufeinander folgend angeordnet sind, vorzugsweise maximal 40 Temperatursensoren (9) pro Segment (14), besonders bevorzugt 10 bis 30 Temperatursensoren (9).

4. Temperatursonde (4) nach einem der Ansprüche 1 bis 3, **dadurch gekennzeichnet, dass** die einzelnen Sensor-Array-Segmente (14) so um den Katheter (3) angeordnet sind, dass sie axial teilweise überlappend um den Katheter anliegen und mit dem Schaft des Katheters (3) an dessen distalem Ende über eine Verbindungsstelle (16) formschlüssig verbunden sind, wobei am distalen Ende die Anschlussbereiche (15) der Sensor-Array-Segmente (14) durch Aussparungen (22) ins Innere des Katheters (3) führen und zwischen Schaft des Katheters (3) und Verbindungsstelle (16) eingepresst sind, und die Leiterbahnen (17) in das Lumen des Katheters (23) führen und dort an Überführungsleiter (18) angeschlossen sind (zur Auswerte- und Steuereinheit).

5. Temperatursonde (4) nach einem der Ansprüche 1 bis 4, **dadurch gekennzeichnet, dass** vier bis sechs Sensor-Array-Segmente (14) rotationssymmetrisch um den Katheter angeordnet sind, vorzugsweise sechs.

6. Temperatursonde (4) nach einem der Ansprüche 1 bis 5, **dadurch gekennzeichnet, dass** jedes Segment (14) einen Messbereich Iₘ mit einer Länge von 200-250 mm aufweist, weiterhin 19-23 mm breit ist und maximal 0,15 mm dick ist, die Gesamtlänge bis zur Verbindung mit Katheterschaft beträgt bis zu 280 mm.

7. Temperatursonde (4) nach einem der Ansprüche 1 bis 6, **dadurch gekennzeichnet, dass** die Sensor-Array-Segmente (14) mehrschichtig sind, wobei eine Trägerschicht (20) als Isolator eine Kopplungsschicht (19) mit eingebetteten Temperatursensoren (9) und Leiterbahnen (17) einschließt, und wobei die jeweiligen Schichten maximal 0,05 mm dick sind.

8. Temperatursonde (4) nach einem der Ansprüche 1 bis 7, **dadurch gekennzeichnet, dass** die röntgendichten Markerbereiche (10) der Sensor-Array-Segmente (14) als weitere Schicht in Balkenform ausgeführt sind.

9. Temperatursonde (4) nach einem der Ansprüche 1 bis 8, **dadurch gekennzeichnet, dass** der Katheter (3) am distalen Ende eine atraumatische Spitze (11) aufweist, die ggf. mit der davor liegenden Verbindungsstelle (16) für die Enden der Sensor-Array-Segmente (14) eine Einheit bildet und formschlüssig mit dem Katheter (3) endet.

10. Temperatursonde (4) nach einem der Ansprüche 1 bis 9, **dadurch gekennzeichnet, dass** die Leiterbahnen (17) Dehnungszonen (25) aufweisen.

11. Temperatursonde (4), nach einem der Ansprüche 1 bis 10, **dadurch gekennzeichnet, dass** sie mit anliegenden Sensor-Array-Segmenten (14) auf dem Katheter (3) maximal einen Durchmesser von 4,5 mm aufweist, wobei der Katheter maximal einen Durchmesser von 2,0 mm besitzt.

12. Temperatursonde (4), nach einem der Ansprüche 1 bis 11, **dadurch gekennzeichnet, dass** die flexiblen Sensor-Array-Segmente (14) am Schaft des Katheters (3) mit einer axial auf dem Katheter (3) beweglichen Einführhülse (26) verbunden sind.

13. Temperatursonde (4), nach einem der Ansprüche 1 bis 11, **dadurch gekennzeichnet, dass** die flexiblen Sensor-Array-Segmente (14) sich distal und proximal in Längsrichtung des Schaftes des Katheters (3) verjüngen, wobei die proximale Verjüngung mit einer auf dem Schaft des Katheters (3) in Längsrichtung zur Katheterspitze beweglichen Hülse (28) über eine weitere Verbindungsstelle (27) verbunden ist, und wobei ggf. eine Begrenzung des Vorschubs für die Hülse (28) auf dem Katheterschaft angeordnet ist.

14. Temperatursonde (4), nach einem der Ansprüche 1 bis 13, **dadurch gekennzeichnet, dass** zwischen dem Katheterschaft und den flexiblen Sensor-Array-Segmenten (14) ein Ballon (29) angeordnet ist.

15. Temperatursonde (4), nach Anspruch 14, **dadurch gekennzeichnet, dass** die Sensor-Array-Segmente (14) einseitig distal vom Ballon mit dem Katheterschaft über eine Verbindungsstelle (16) oder zweiseitig, distal vom Ballon mit dem Katheterschaft und proximal vom Ballon mit einer auf dem Schaft des Katheters (3) in Längsrichtung zur Katheterspitze beweglichen Hülse (28) über eine weitere Verbindungsstelle (27) verbunden ist.

## Claims

1. A temperature probe (4) for inserting into the esophagus (2), **characterized in that** said temperature probe comprises at least two flexible flat sensor array segments (14) disposed rotationally symmetrically around a catheter (3) and the surfaces thereof taper at least towards the distal end of the catheter (3) so that said surfaces form connection regions (15) connected to the catheter (3) at the distal end, the sensor array segments (14) each comprising one or more temperature sensors (9) connected via conductor tracks (17), the temperature sensors (9) being functionally contactable by the mucosal surface of the esophagus (2), so that a temperature on the mucosal surface is detectable via the temperature sensors (9).

2. The temperature probe (4) according to claim 1, **characterized in that** the sensor array segments (14) each comprise distal and proximal radiopaque marker regions, the temperature sensors (9) being disposed between said marker regions, said temperature sensors (9) defining a measuring range Iₘ from marker to marker.

3. The temperature probe (4) according to claim 1 or 2, **characterized in that** the temperature sensors (9) in the sensor array segments (14) are disposed meandering or successively in a row from the proximal to the distal marker, preferably a maximum of 40 temperature sensors (9) per segment (14), particularly preferably 10 to 30 temperature sensors (9).

4. The temperature probe (4) according to any one of claims 1 to 3, **characterized in that** the individual sensor array segments (14) are disposed around the catheter (3) so as to fit around the catheter in a axially partially overlapping manner and are connected in a form-fitting manner to the shaft of the catheter (3) at the distal end thereof via a connection point (16), wherein the connection regions (15) of the sensor array segments (14) lead through recesses (22) into the interior of the catheter (3) at the distal end and are pressed between the shaft of the catheter (3) and the connection point (16), and guide the conductor tracks (17) into the lumen of the catheter (23) and are connected there to crossover conductors (18) (to the evaluation and control unit).

5. The temperature probe (4) according to any one of claims 1 to 4, **characterized in that** four to six, preferably six, sensor array segments (14) are disposed rotationally symmetrically around the catheter.

6. The temperature probe (4) according to any one of claims 1 to 5, **characterized in that** each segment (14) has a measuring range Iₘ having a length of 200-250 mm, is further 19-23 mm wide and has a maximum thickness of 0.15 mm, the total length up to the connection to catheter shaft being up to 280 mm.

7. The temperature probe (4) according to any one of claims 1 to 6, **characterized in that** the sensor array segments (14) are multi-layered, wherein a carrier layer (20), as an insulator, encloses a coupling layer (19) having embedded temperature sensors (9) and conductor tracks (17), and wherein the respective layers are a maximum of 0.05 mm thick.

8. The temperature probe (4) according to any one of claims 1 to 7, **characterized in that** the radiopaque marker regions (10) of the sensor array segments (14) are designed as a further layer in the form of a beam.

9. The temperature probe (4) according to any one of claims 1 to 8, **characterized in that** the catheter (3) comprises an atraumatic tip (11) at the distal end, said tip optionally forming a unit with the connection point (16) in front of said tip for the ends of the sensor array segments (14) and ending in a form-fitting manner with the catheter (3).

10. The temperature probe (4) according to any one of claims 1 to 9, **characterized in that** the conductor tracks (17) comprise expansion zones (25).

11. The temperature probe (4) according to any one of claims 1 to 10, **characterized in that** said temperature probe has a maximum diameter of 4.5 mm with adjacent sensor array segments (14) on the catheter (3), wherein the catheter has a maximum diameter of 2.0 mm.

12. The temperature probe (4) according to any one of claims 1 to 11, **characterized in that** the flexible sensor array segments (14) on the shaft of the catheter (3) are connected to an insertion sleeve (26), said sleeve being axially displaceable on the catheter (3).

13. The temperature probe (4) according to any one of claims 1 to 11, **characterized in that** the flexible sensor array segments (14) taper distally and proximally in the longitudinal direction of the shaft of the catheter (3), wherein the proximal taper is connected via a further connection point (27) to a sleeve (28), said sleeve being displaceable in the longitudinal direction on the shaft of the catheter (3) to the catheter tip, and optionally a limitation of the feed for the sleeve (28) is disposed on the catheter shaft.

14. The temperature probe (4) according to any one of claims 1 to 13, **characterized in that** a balloon (29) is disposed between the catheter shaft and the flexible sensor array segments (14).

15. The temperature probe (4) according to claim 14, **characterized in that** the sensor array segments (14) are connected distally on one side from the balloon to the catheter shaft via a connection point (16) or are connected distally on two sides from the balloon to the catheter shaft and proximally from the balloon to a sleeve (28), said sleeve being displaceable in the longitudinal direction to the catheter tip on the shaft of the catheter (3) via a further connection point (27).

## Revendications

1. Sonde de température (4) destinée à être introduite dans l'œsophage (2), **caractérisée en ce qu'**elle présente au moins deux segments (14) de réseaux de capteurs plats souples qui sont disposés en symétrie de rotation autour d'un cathéter (3) et dont les surfaces se rétrécissent au moins vers l'extrémité distale du cathéter (3), de sorte que celles-ci forment des zones de connexion (15), qui sont reliées au cathéter (3) à l'extrémité distale, dans laquelle les segments de réseaux de capteurs (14) comportent chacun un ou plusieurs capteurs) de température (9) qui sont reliés via des pistes conductrices (17), dans laquelle les capteurs de température (9) peuvent être mis en contact fonctionnel avec la surface de la muqueuse de l'œsophage (2) de sorte qu'une température à la surface de la muqueuse peut être détectée via les capteurs de température (9).

2. Sonde de température (4) selon la revendication 1, **caractérisée en ce que** les segments (14) de réseaux de capteurs présentent chacun des zones de marqueurs radio-opaques distales et proximales entre lesquelles sont disposés les capteurs de température (9) qui définissent une zone de mesure Iₘ de marqueur à marqueur.

3. Sonde de température (4) selon la revendication 1 ou 2, **caractérisée en ce que** les capteurs de température (9) sont disposés les uns après les autres dans les segments (14) de réseaux de capteurs en serpentin ou en série(s), du marqueur proximal au marqueur distal, de préférence au maximum 40 capteurs de température (9) par segment (14), de manière particulièrement préférée 10 à 30 capteurs de température (9).

4. Sonde de température (4) selon l'une quelconque des revendications 1 à 3, **caractérisée en ce que** les différents segments (14) de réseaux de capteurs sont disposés autour du cathéter (3) de sorte qu'ils reposent autour du cathéter en chevauchement axial partiel et sont reliés, via un point de connexion (16), par complémentarité de forme, à la tige du cathéter (3) à son extrémité distale, dans laquelle, à l'extrémité distale, les zones de raccordement (15) des segments (14) de réseaux de capteurs mènent à l'intérieur du cathéter (3) à travers des évidements (22) et sont insérées en force entre la tige du cathéter (3) et le point de raccordement (16), et les pistes conductrices (17) mènent dans le lumen du cathéter (23) et y sont connectées à des conducteurs de transfert (18) (vers l'unité d'exploitation et de commande).

5. Sonde de température (4) selon l'une quelconque des revendications 1 à 4, **caractérisée en ce que** quatre à six, de préférence six, segments (14) de réseaux de capteurs sont disposés en symétrie de rotation autour du cathéter.

6. Sonde de température (4) selon l'une quelconque des revendications 1 à 5, **caractérisée en ce que** chaque segment (14) présente une zone de mesure lm ayant une longueur de 200-250 mm, est en outre de 19-23 mm en largeur et au maximum de 0,15 mm en épaisseur, la longueur totale jusqu'au raccordement avec la tige de cathéter vaut jusqu'à 280 mm.

7. Sonde de température (4) selon l'une quelconque des revendications 1 à 6, **caractérisée en ce que** les segments (14) de réseaux de capteurs sont multicouches, dans laquelle une couche de support (20) servant d'isolant entoure une couche de couplage (19) avec des capteurs de température (9) et des pistes conductrices (17) incorporés, et dans laquelle les couches respectives ont une épaisseur maximale de 0,05 mm.

8. Sonde de température (4) selon l'une quelconque des revendications 1 à 7, **caractérisée en ce que** les zones de marqueurs radio-opaques (10) des segments (14) de réseaux de capteurs sont réalisées sous la forme d'une couche supplémentaire en forme de barre.

9. Sonde de température (4) selon l'une quelconque des revendications 1 à 8, **caractérisée en ce que** le cathéter (3) présente à l'extrémité distale une pointe atraumatique (11) qui, le cas échéant, forme une unité avec le point de raccordement (16) disposé devant elle pour les extrémités des segments (14) de réseaux de capteurs et se termine en complémentarité de forme avec le cathéter (3).

10. Sonde de température (4) selon l'une quelconque des revendications 1 à 9, **caractérisée en ce que** les pistes conductrices (17) présentent des zones d'expansion (25).

11. Sonde de température (4) selon l'une quelconque des revendications 1 à 10, **caractérisée en ce qu'**elle présente un diamètre maximal de 4,5 mm avec des segments (14) de réseaux de capteurs adjacents sur le cathéter (3), dans laquelle le cathéter présente un diamètre maximal de 2,0 mm.

12. Sonde de température (4) selon l'une quelconque des revendications 1 à 11, **caractérisée en ce que** les segments (14) de réseaux de capteurs flexibles sur la tige du cathéter (3) sont reliés à une douille d'insertion (26) mobile axialement sur le cathéter (3).

13. Sonde de température (4) selon l'une quelconque des revendications 1 à 11, **caractérisée en ce que** les segments (14) de réseaux de capteurs flexibles se rétrécissent distalement et proximalement dans la direction longitudinale de la tige de cathéter (3), dans laquelle le rétrécissement proximal est relié via un autre point de raccordement (27) à une douille (28) mobile sur la tige de cathéter (3), dans la direction longitudinale vers la pointe du cathéter, et dans laquelle une limitation de l'avancement pour la douille (28) est éventuellement disposée sur la tige de cathéter.

14. Sonde de température (4) selon l'une quelconque des revendications 1 à 13, **caractérisée en ce qu'**un ballon (29) est disposé entre la tige de cathéter et les segments (14) de réseaux de capteurs flexibles.

15. Sonde de température (4) selon la revendication 14, **caractérisée en ce que** les segments (14) de réseaux de capteurs sont reliés d'un seul côté, à distance du ballon, à la tige de cathéter via un point de raccordement (16), ou de deux côtés, à distance du ballon, à la tige de cathéter, et, à proximité du ballon, à une douille (28) mobile sur la tige de cathéter (3) dans le sens longitudinal, vers la pointe du cathéter, via un point de raccordement (27) supplémentaire.
